# EUROPÄISCHE PATENTSCHRIFT

(11) **EP 1 951 134 B1**
(45) Veröffentlichungstag und Bekanntmachung des Hinweises auf die Patenterteilung: **16.03.2016**
(21) Anmeldenummer: 06791323.6
(22) Anmeldetag: 30.08.2006
(51) Int. Cl.: A61B 17/80

(54) **KNOCHENPLATTE MIT MINDESTENS EINER SCHRAUBE ZUR WINKELSTABILEN FIXATION**
BONE PLATE COMPRISING AT LEAST ONE SCREW TO BE FIXED AT A STABLE ANGLE
LAME OSSEUSE PRESENTANT AU MOINS UNE VIS PERMETTANT UNE FIXATION A ANGLE STABLE

(30) Priorität: 01.09.2005 DE 202005013868 U
(43) Veröffentlichungstag der Anmeldung: 06.08.2008
(73) Patentinhaber: Merete Medical GmbH, 12247 Berlin (DE)
(72) Erfinder: ANAPLIOTIS, Emmanuel, 14195 Berlin (DE); KRANZ, Curt, 14163 Berlin (DE)
(74) Vertreter: Hannig, Wolf-Dieter
(86) Internationale Anmeldenummer: PCT/DE2006/001508
(87) Internationale Veröffentlichungsnummer: WO 2007/025520

(56) Entgegenhaltungen:
- WO-A-97/09000
- FR-A- 2 667 913
- FR-A- 2 739 151
- US-A1- 2004 073 218
- "Orthopaedic Product News" [Online] August 2005 (2005-08), , XP002415364 Gefunden im Internet: URL:http://www.orthoworld.com/us_opn-2005- 08.pdf> [gefunden am 2007-01-17] Seite 30 Hallux Valgus Correction With A Low Profile Locking Plate

## Beschreibung

Die Erfindung betrifft eine Knochenplatte mit mindestens einer Schraube zur winkelstabilen Fixation der im Oberbegriff des Anspruchs 1 genannten Art.

Eine derarige Knochenplatte ist als "Metafix" - Implantat der Firma Merete Medical GmbH, Bertlin bekannt.
Es dient zur Fixierung bei der Beseitigung der so genannten Halux-Valgus-Deformität. Hierbei kommt es darauf an, dass die zu fixierenden Zehenbereiche durch die Platte richtungsstabil gehalten werden. Dazu weisen die Schrauben nicht nur ein Knochenschraubengewinde zum Fixieren im Knochen in ihrem unteren Bereich auf, sondern auch der Kopfbereich ist noch mit einem Außen-Feingewinde versehen, welche in ein entsprechendes Innengewinde des Schraubloches der Platte eingreift, wenn die Schraube beim Eindrehen die entsprechende Position erreicht hat. Hiermit ist sie dann richtungsstabil ausgerichtet. Um sie auch noch in Schraubrichtung zu fixieren wird sie anschließend - wie für eine Schraube mit Kopf üblich - festgezogen, wobei sich das konische Außengewinde des Kopfes im zylindrischen Innengewinde der Platte festsetzt.

Nachteilig ist dabei, dass der Bereich des Festsetzens des Gewindes - und damit die Endposition der Schraube genau festgelegt ist, so dass der Einstellbereich der Schraube - und auch derjenige der Platte - begrenzt ist.
Ein entsprechendes Implantat ist auch aus dem US-Patent 3741205 bekannt, wobei hier allerdings glatte Pins anstelle von Schrauben verwendet werden, so dass die Verhältnisse bezüglich des Einschraubvorgangs vereinfacht sind.

Weiterhin ist aus der veröffentlichten US-Patentanmeldung US2004/0073218A1 ein orthopädisches Plattensystem bekannt, bei dem eine orthopädische Schraube mit einem ein Gewinde aufweisenden Kopf in unterschiedlichen Richtungen einschraubbar sein soll. In der Platte ist aber für den Kopf kein entsprechendes Gegengewinde vorhanden, so dass dieser dort keinen festen Halt findet. Außerdem schneidet sich das Gewinde des Schraubenkopfes in der Platte unter Erzeugung von Materialspänen ein, so dass eine Gefährdung des Patienten die Folge ist.

Eine aus dem US-Patent 6306140B1 bekannte Knochenschraube weist ebenfalls einen mit einem Gewinde versehenen Kopf auf. Diese Schraube ist aber nicht zur Anwendung mit Knochenplatten bestimmt.

Der Erfindung liegt die Aufgabe zu Grunde, eine Knochenplatte der eingangs genannten Art derart weiterzubilden, dass der Einstellbereich der Schraube vergrößert ist, so dass ein Festsitzen der Schraube nicht abrupt eintritt, sondern in einem größeren deren Festsitz gegeben ist. Damit kann die Schraube weiter angezogen und der Sitz bzw. die Kompression der Platte verbessert werden, ohne dass die Schraube über- oder abgedreht wird.

Diese Aufgabe wird bei einer Knochenplatte der eingangs genannten Art durch die im kennzeichnenden Teil des Anspruchs 1 angegebenen Maßnahmen gelöst.

Die Vorteile der Knochenplatte gemäß der Erfindung liegen insbesondere darin, dass der an das Außengewinde der Platte anschließende Bereich sich derart elastisch verformt, dass er sich an den Außendurchmesser des Konus des Innengewindes anpasst - und zwar über den ganzen Weg des Schraubendurchgangs. Die Fixationsschraube kann damit mit ihrem Kopf vollständig durch das Loch in der Platte "hindurch geschraubt" werden, wobei der Kopf durch die elastische Spannung in jeder Position einerseits drehsicher gehalten ist, andererseits die Platte aber auch nicht in ihren plastischen Bereich irreversibel verformt, d.h. überdehnt wird. Auf diese Weise lässt sich erreichen, dass die Schraube feinfühlig genau so weit eingedreht werden, wie es erforderlich ist, die Platte fest am Knochen anliegend zu fixieren, wobei gleichzeitig sichergestellt werden kann, dass der Schraubenkopf in seinen erhabensten Bereichen auch die Platte nicht überragt, so dass die fixierte Platte insgesamt - mit ihren Schrauben - eine im Wesentlichen sich an den Knochen ohne wesentliche Erhebungen glatt anschmiegende Einheit bildet.

Die Abmessungen der die Schraublöcher umgebenden Stege werden dabei gerade so gewählt, das die für den Durchlass des Schraubenkopfes notwendige Dehnung, die der wirksamen Umfangsdifferenz des beim Einschrauben wirksamen Konus des Kopfes entspricht, keine Dehnung des Materials in den plastischen Bereich hinein bewirkt. Dies kann mittels des üblichen Spannungs-Dehnungs-Diagramms unter Berücksichtigung der Schraubenkopf/Schraubloch-Geometrie für die beteiligten Werkstoffe leicht ermittelt werden.

Wenn dann, entsprechend einer bevorzugten Ausführung der das Durchgangsloch umgebende Dehnungsbereich mindestens teilweise als Steg oder Teil eines Ringes ausgebildet ist, lassen sich in bevorzugter Weise diese Bereich so gestalten, dass sich hier die Dehnung konzentriert, so dass die Dimensionierung in definierter Weise in Anpassung an den Konus des Schraubenkopfes erfolgen kann.

Dabei ist es insbesondere günstig, wenn der Steg oder Ring eine konstante Breite aufweist, an den beidseitig ein Bereich der Platte mit sich vergrößernder Stegbreite anschließt, ist eine weitere Vereinfachung bei der Ermittlung der für die gewünschten Dehnungen notwendigen geometrischen Dimensionierung möglich.

Bevorzugt weist ein Durchgangsloch mindestens zwei Bereiche im Wesentlichen konstanter Breite aufweist, an den sich beidseitig ein Bereich der Platte mit vergrößerter Stegbreite anschließt. Da die Platte eine im Wesentlichen konstante Dicke aufweist, ist also der Querschnitt insgesamt ebenfalls im Wesentlichen über die Länge des stegartigen bzw. teilringförmigen Bereichs gleich bleibend. Auf diese Weise ergibt sich einerseits eine besonders günstige Gestaltung der Knochenplatte, bei der sich die zum Andruck auf den Knochen bestimmten Bereiche getrennt von den die Schraubenköpfe führenden Bereichen dimensionieren lassen, so dass eine vorteilhafte Funktionstrennung die Folge ist. Darüber hinaus verteilen sich die Dehnungsbereiche jeweils eines Schraublochs der Platte definiert auf jeweils zwei Stegbereiche, welche sich die Dehnung "aufteilen", so dass die Verformung des angrenzenden Andruckbereichs beschränkt bleiben kann. Es sind also einerseits die Andruckflächen, welche wirksam an dem Knochen aufliegen maximiert, während die die Dehnungsbereiche hauptsächlich die tangential in Bezug auf den Schraubenkopf wirkenden Dehnspannungen aufnehmen und damit definiert belastet sind, so dass eine plastische Materialverformung und in jedem Fall ein Bruch des Stegs wegen Überlastung sicher vermieden sind.

Dabei ist die Platte vorteilhafter Weise so gestaltet, dass der Andruckbereich gegenüber dem das Schraubloch umgebenden, die Stege oder Ringteile aufweisenden Bereich eine Einschnürung bildet. Damit können die Stege oder Ringteile derart gestaltet werden, dass sie in Bezug auf die als Andruckflächen wirksamen Bereiche eine maximale Länge besitzen, so dass die relative Dehnung der Dehnungsbereiche minimiert ist. Auf diese Weise lässt sich in optimaler Weise die Forderung erfüllen, dass die Dehnungszonen sich über den vorgesehenen Bereich der elastischen Verformung im Spannungs-DehnungsDiagramm hinaus ausdehnen.

In günstiger Weise bestehen dabei die Knochenschraube und/oder die Platte aus einer köperverträglichen Titanlegierung. Das Spannungs-DehnungsDiagramm der üblichen Titan-Legierungen weist dabei einen Bereich sich verringernder Steigung auf, der über einen Plateaubereich in einen abfallenden Verlauf übergeht, der das plastische Verhalten kennzeichnet. Gemäß einer bevorzugten Ausführungsform der Erfindung wird die zum Durchlass des konischen Schraubenkopfes notwendige Dehnung des Materials der Platte im Bereich der das Schraubloch umgebenden Steg- oder Ringbereiche derart gewählt, dass ein Dehnungsbereich im oberen Bereich des Anstiegs in dem Teil der Kurve gewählt, in dem der Übergang zur horizontalen im Gipfelbereich der Kurve beginnt.

Während generell auch das Schraubloch eine Verjüngung aufweisen kann, die in Einschraubrichtung der Knochenschraube lediglich geringer dimensioniert sein muss als die durch die Steigung des Konus des Schraubenkopfes bedingte Verjüngung, ist bei einer optimal vereinfachten Ausführungsform er Erfindung lediglich der Schraubenkopf konisch verjüngt, während das Feingewinde des Schraubloches zylindrisch ausgebildet ist.

Weiterhin vorteilhaft bei der Erfindung ist, wenn die Steigung des Feingewindes am Schraubenkopf geringer ist als die Steigung des Knochengewindes am Schaft, welche beim Einschrauben in den Knochen wirksam ist. Auf diese Weise lässt sich erreichen, dass mit dem hindurch treten des Schraubenkopfes durch das Schraubloch der Platte, diese durch den Schraubenkopf weiterhin zum Knochen hin gezogen wird, so dass die gewünschte Kompression sicher erreichbar ist. Die lässt sich durch das Feingewinde in der Platte feinfühlig einstellen, wobei gleichzeitig sichergestellt ist, dass durch die den Schraubenkopf umgebende Zugspannung des stegförmigen Materials des Lochbereichs eine gute Verdrehsicherung ausgeübt wird, die sich über einen längeren Schraubweg erstreckt. Dies steht in vorteilhaftem Gegensatz zu den üblichen Schraubverbindungen der Technik, welche im festgezogenen Zustand lediglich eine Verdrehung um einen äußerst geringfügigen Drehwinkel benötigen, um in den losen, leicht verdrehbaren Zustand zu gelangen.

Die erfindungsgemäße Lösung weist darüber hinaus noch den Vorteil auf, dass der Schraubenkopf derart tief abgesenkt werden kann, dass er nicht über die Plattenoberfläche hinaus vorsteht.'Er also kein störendes Hindernis in Bezug auf die Anschmiegfähigkeit der Platte bildet und insbesondere auch nicht im implantierten Zustand ertast- oder fühlbar ist. Das wird gemäß einer vorteilhaften Weiterbildung der Erfindung dadurch erreicht, dass Gewindegrund des Knochengewindes der Schraube in der Nachbarschaft des Kopfes sich, insbesondere konisch, erweitert und im Wesentlichen an den Gewindegrund des benachbarten Feingewindes am Kopf anschließt. Auf diese Weise wird beim Eindrehen der Schraube im Kopfbereich ein gewisser Platz für das Feingewinde im Knochen geschaffen, so dass der Kopf in seiner Bewegung beim Einschrauben nicht durch die Knochenoberfläche begrenzt wird. Auf diese Weise kann er in seiner Höhe so eingestellt werden, dass seine Deckfläche mit der Oberfläche der Knochenplatte abschließt.

Dazu ist es insbesondere auch - entsprechend einer anderen vorteilhaften Weiterbildung der Erfindung förderlich, wenn der Schraubenkopf einen Innensechskant aufweist.

Nachfolgend wird eine bevorzugte Ausführungsform der Erfindung anhand der Zeichnungen näher erläutert. Es zeigen:
Fig. 1 das Ausführungsbeispiel der erfindungsgemäßen Knochenplatte in Draufsicht,
Fig. 2 einen Schnitt durch das Ausführungsbeispiel gemäß Fig. 1 entlang der Schnittlinie II in Fig. 1,
Fig. 3 einen weiteren Schnitt durch das Ausführungsbeispiel gemäß Fig 1 entlang der Schnittlinie III in Fig. 1 mit einer mit der dargestellten Knochenplatte in Wechselwirkung befindlichen Knochenschraube, die ebenfalls im Schnitt dargestellt ist sowie
Figur 4 eine perspektivische Darstellung des Ausführungsbeispiels der erfindungsgemäßen Platte mit eingedrehten Schrauben in perspektivischer Darstellung.

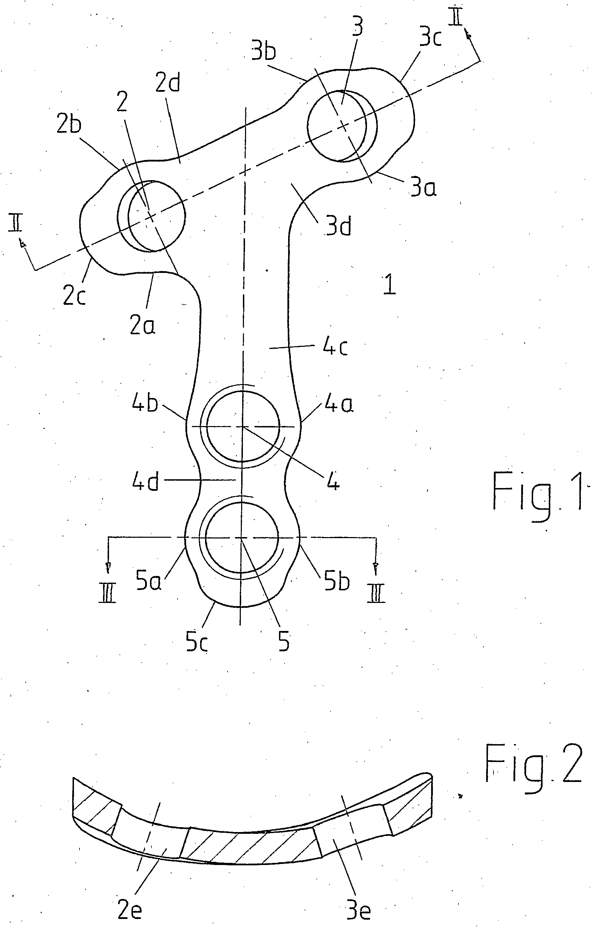

Bei dem in Figur 1 dargestellten Ausführungsbeispiel der Erfindung ist eine aus einer körperverträglichen Titanlegierung bestehende Knochenplatte 1 mit Schraublöcher bildenden Durchgangslöchern 2 bis 4 versehen. Die Schraublöcher 2 bis 4 sind jeweils mit einem metrischen Innengewinde versehen, wie es jeweils durch eine die Schraublöcher 2 bis 5 umgebende ringförmige Linie angedeutet (und weiter an Hand der Schnittbilder näher beschrieben) ist. Die Schraublöcher sind zum Teil umgeben von jeweils zwei steg- bzw. teilringförmigen Bereichen 2a und 2b, 3a und 3b, 4a und 4b sowie 5a und 5b. Diese das jeweilige Durchgangsloch umgebenden Bereiche sind derart elastisch ausgebildet ist, dass das Material der Knochenplatte durch die sich infolge der durch das Einschrauben des Kopfes einer Knochenschraube ergebenden Dehnung nicht in den plastischen Bereich verformt wird, so dass durch die Wechselwirkung des Außengewindes des Kopfes der Knochenschraube mit dem Innengewinde der Platte über die gesamte Länge des Gewindes unter Aufrechterhaltung der Drehsicherung in keiner Position der Knochenschraube ein Überdehnen des das Durchgangsloch umgebenden Bereichs eintritt. Diese als Stege ausgebildeten teilringförmigen Bereiche weisen dabei in der Draufsicht eine konstante Breite auf. Da auch die Plattendicke im wesentlichen konstant ist, haben die Stege einen im wesentlichen gleich bleibenden Querschnitt, was dazu führt, dass die differentiellen Dehnungen bei Einschrauben der Knochenschraube über die Länge des steg- oder teilringförmigen Bereichs gleich bleibend sind.

Beidseitig schließt an die steg- bzw. teilringförmigen Bereiche 2a und 2b, 3a und 3b, 4a und 4b sowie 5a und 5b jeweils eine Zone 2c und 2d, 3c und 3d, 4c und 4d sowie 4d und 5c an, welche einen eingeschnürten Bereich der Platte bildet, wobei diese Zonen im Hinblick auf die steg- oder teilringförmigen Bereiche 2a und 2b, 3a und 3b, 4a und 4b sowie 5a und 5b aber Querschnittserweiterungen bilden. Der Breiuchg 4d stellt dabei einen solchen Bereich sowohl im Hinblick auf die Bohrung 4 als auch die Bohrung 5 dar, da er beiden benachbart ist.

In Figur 2c ist ein Schnitt der Platte gemäß der Schnittlinie II-II in Figur 1 dargestellt.
Es sind die Bohrungen 2 und 3 mit ihren Gewindebohrungen 2e und 3e wiedergegeben. Es ist ersichtlich, dass die Platte 1 im Profil gewölbt ist, wobei die Wölbung entsprechend der Knochenoberfläche gewählt ist. Die Gewindebohrungen sind damit senkrecht zur Knochenoberfläche gerichtet, so dass sie damit radial in Bezug auf den Bogen der Wölbung stehen.

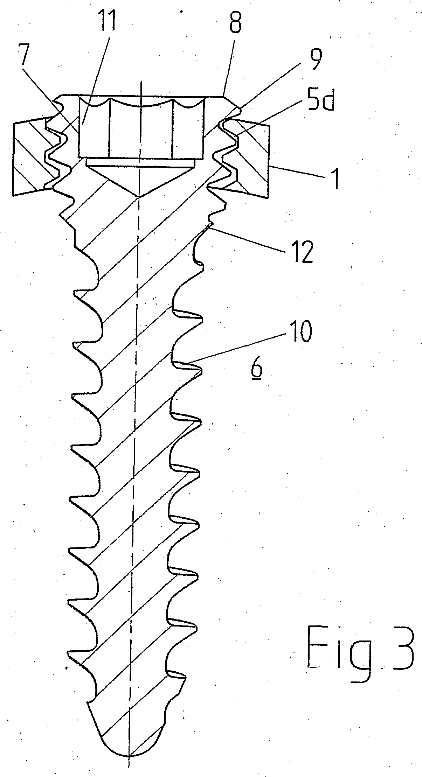

In Figur 3 ist ein weiterer Schnitt der Platte gemäß Schnittlinie 111-111 in Figur 1 dargestellt. In die Gewindebohrung 5e der Bohrung 5 ist eine Knochenschraube 6 eingefügt, die ebenfalls aus Titan besteht. Sie weist einen Kopf 7 mit einem Innensechskant 11 auf, der mit eine metrischen Außengewinde 9 versehen ist, welches sich aber im Gegensatz zum zylindrischen Innengewinde 5d der Gewindebohrung 5 zum oberen Ende 8 hin konisch erweitert.. Am unteren Ende weist die Schraube 6 ein Knochenschraubengewinde 10 auf, dessen Steigung größer ist als die des Außengewindes 9 am Schraubenkopf 7.

Es ist ersichtlich, dass beim Einschrauben der Knochenschraube 6 diese zunächst mittels des Knochenschraubengewindes 10 in den Knochen vordringt und sich dort fixiert bis das Außengewinde 9 des Kopfes 7 in das Innengewinde 5d der Platte 1 eingreift. Da die Steigung des Innengewindes 5d geringer ist als die des Knochenschraubengewindes 10, beginnt die Platte sich mit dem weiteren Eindrehen der Schraube 6 an den bei der Anwendung benachbarten Knochen anzulegen.
Da sich das Außengewinde 9 des Kopfes 7 nach oben erweitert, geraten die diesen umgebenden Stegbereiche 5a und 5b (siehe Fig. 1) unter Spannung und beginnen sich zu expandieren. Die sich damit aufbauende Zugspannung bildet eine Sicherung für den Schraubenkopf 7 gegen unbeabsichtigte Verdrehungen. Da der das Durchgangsloch bildende Bohrung 5 umgebende Bereich derart elastisch ausgebildet ist, dass das Material der Knochenplatte durch die sich infolge der durch das Einschrauben des Kopfes der Knochenschraube ergebenden Dehnung nicht in den plastischen Bereich verformt wird, ist eine Überdehnung der Steg- bzw. teilringförmigen Bereiche vermieden. Auf diese Weise ist sichergestellt, dass durch die Wechselwirkung des Außengewindes 9 des Kopfes 7 der Knochenschraube 6 mit dem Innengewinde- 5e der Platte über die gesamte Länge des Gewindes unter Aufrechterhaltung der Drehsicherung in keiner Position der Knochenschraube 6 ein Überdehnen des das Durchgangsloch 5 umgebenden Bereichs eintritt.

Die Schraube 6 kann jetzt so weit hineingedreht werden, dass der Kopf 7 bündig mit der Oberfläche der Platte 1 abschließt und insoweit diese nicht störend überragt. Die Schraube 6 kann ohne weiteres auch ganz durch die Platte 1 hindurchgeschraubt werden, ohne dass diese bleibend geschädigt wird. Insoweit ist also auch kein Überdrehen der Schraube möglich, was bei konventionellen Verschraubungen meist mit einer Zerstörung des Gewindes und einer im Implantationsbereich unbedingt zu vermeidenden Bildung von Metallspänen verbunden wäre. Dabei wird der Schraubenkopf 7 derart tief abgesenkt, dass er nicht über die Plattenoberfläche hinaus vorsteht. Er bildet also kein störendes Hindernis in Bezug auf die Anschmiegfähigkeit der Platte und ist insbesondere auch nicht im implantierten Zustand ertast- oder fühlbar. Das wird dadurch erreicht, dass Gewindegrund des Knochengewindes der Schraube in einem Bereich 12 in der Nachbarschaft des Kopfes sich, insbesondere konisch, erweitert und im Wesentlichen an den Gewindegrund des benachbarten Feingewindes am Kopf anschließt. Auf diese Weise wird beim Eindrehen der Schraube im Kopfbereich ein gewisser Platz für das Feingewinde im Knochen geschaffen, so dass der Kopf in seiner Bewegung beim Einschrauben nicht durch die Knochenoberfläche begrenzt wird. Damit kann er in seiner Höhe insbesondere so eingestellt werden, dass seine Deckfläche mit der Oberfläche der Knochenplatte abschließt.

Die Schraube 6 weist zum Einschrauben in ihrem Kopf 7 einen Innensechskant 11 auf.

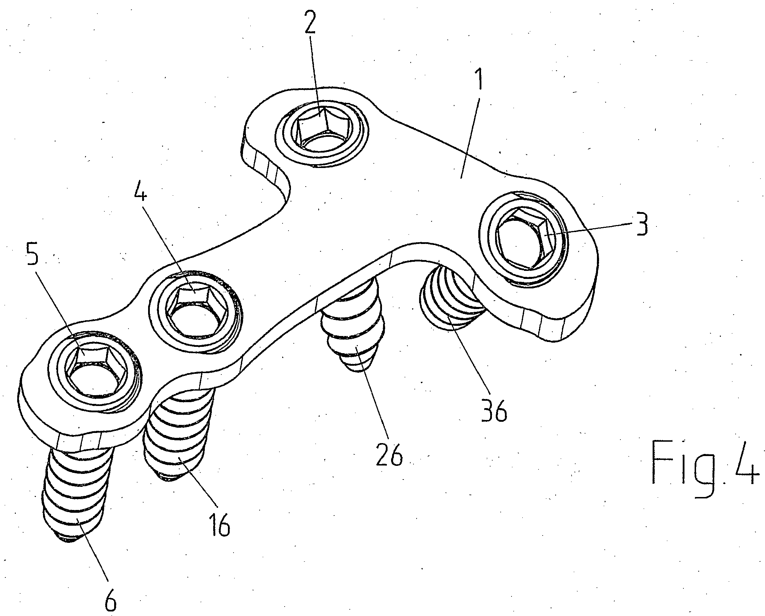

In Figur 4 ist die vollständige Knochenplatte 1 mit Schrauben 6, 16, 26 und 36 in perspektivischer Ansicht wiedergegeben. Es ist ersichtlich, dass die Schrauben in ihrer Richtung durch die Wölbung der Oberfläche der Platte 1 bestimmt und durch die Innengewinde der Löcher in Wechselwirkung mit den Schraubenköpfen stabil geführt werden. Die übrigen Elemente sind über ihre Bezugszeichen identifizierbar und weiter oben beschrieben. In die Platte 1 sind dabei die Schrauben 6, 16, 26 und 36 in der jeweils durch das Gewinde in der Platte vorgegebenen Richtung soweit eingeschraubt, dass auch das Gewinde im Bereich ihrer Köpfe in das Innengewinde der Platte eingreift. Hierbei sind die Schrauben über ihre Innensechskante 2 bis 4 jeweils in einem weiten Bereich einstellbar, wobei der Knochen der durch das Knochengewinde der Schraube erfasst wird, beim weiteren Eindrehen der Schraube an die Platte herangezogen wird, wenn - gemäß der entsprechenden vorteilhaften Ausführung - das Knochengewinde am Schaft eine größere Steigung aufweist, als das Gewinde der Schraube in ihrem Kopfbereich. Einzusätzliches Verklemmen der Schraube im Sinne einer Verdrehsicherung tritt ein, wenn das Innen- und das Außengewinde der Schraube insoweit von einander abweichen, dass diese eine geringfügige Steigungs- oder Neigungsabweichung besitzen. Bei der Neigung ist hierbei die Ausgestaltung als zylindrisches bzw. konisches Gewinde mit unterschiedlicher Konizität gemeint.

Die Erfindung ist nicht auf das vorbeschriebene Ausführungsbeispiel beschränkt und an einer Vielzahl von Implantaten und Knochenplatten anwendbar, wenn es darauf ankommt, eine Knochenschraube einerseits in unterschiedlichen Positionen gegen unbeabsichtigtes Verdrehen gesichert zu halten und andererseits den Schraubenkopf derart bündig mit der Oberfläche des Implantats positionierbar zu halten, dass dieser nicht störend vorsteht.

## Patentansprüche

1. Knochenplatte mit mindestens einer Schraube zur winkelstabilen Fixation, insbesondere als Hallux-Valgus-Platte, mit mindestens einem Durchgangsloch (2,3,4,5) und mindestens einer Knochenschraube (6), welche im Kopfbereich ein Außengewinde (9) aufweist, das sich in Einschraubrichtung stärker verjüngt als ein als Innengewinde (5d) ausgebildetes Gegengewinde des Durchgangslochs, so dass sich der Kopf (7) der Knochenschraube (6) beim Einschrauben zur Drehsicherung zwischen Außengewinde (9) des Kopfes (7) und Innengewinde (5d) der Platte (1) richtungsstabil verklemmt,
**dadurch gekennzeichnet,**
**dass** die Knochenplatte (1) in mindestens einem an das Durchgangsloch (2,3,4,5) angrenzenden Bereich (2a;2b;3a;3b;4a;4b) derart elastisch ausgebildet ist, dass das Material der Knochenplatte (1) durch die sich infolge der durch das Einschrauben des Kopfes (7) der Knochenschraube (6) ergebenden Dehnung nicht in den plastischen Bereich hinein verformt wird, so dass durch die Wechselwirkung des Au0ßengewindes (9) des Kopfes (7) der Knochenschraube (6) mit dem Innengewinde der Platte über die gesamte Länge der Gewinde (5d, 9) unter Aufrechterhaltung der Drehsicherung in keiner Position der Knochenschraube (6), wenn sie ganz durch die Platte (1) hindurch geschraubt wird, ein Überdehnen des das Durchgangsloch (2,3,4,5) umgebenden Bereichs eintritt.

2. Knochenplatte nach Anspruch 1, **dadurch gekennzeichnet, dass** der an das Durchgangsloch (2,3,4,5) angrenzende Bereich (2a,2b,3a,3b, 4a,4b) mindestens teilweise als Steg oder Teil eines Ringes ausgebildet ist.

3. Knochenplatte nach Anspruch 2, **dadurch gekennzeichnet, dass** der an das Durchgangsloch (2,3,4,5) angrenzende Bereich (2a,2b,3a,3b,4a,4b) in der Draufsicht eine im Wesentlichen konstante Breite bzw. im Schnitt eine im Wesentlichen konstanten Materialquerschnitt aufweist, an den beidseitig ein Bereich mit sich vergrößernder Stegbreite anschließt, der aber in Bezug auf die Platte in der Draufsicht eine Einschnürung darstellt.

4. Knochenplatte nach Anspruch 3, **dadurch gekennzeichnet, dass** der an das Durchganghsloch (2,3,4,5) angrenzende Bereich (2a,2b,3a,3b,4a,4b) mindestens aus zwei Bereichen konstanter Breite besteht, an den sich beidseitig ein Bereich der Platte mit vergrößerter Stegbreite (23c,2d,3c,3d,4c,4d,5c) anschließt.

5. Knochenplatte nach Anspruch 2, **dadurch gekennzeichnet, dass** der an das Durchgangsloch 2,3,4,5) angrenzende Bereich (2a,2b,3a,3b.4a,4b) sich ausschließlich auf den steg- oder ringförmigen Bereich konzentriert, der in Bezug auf den benachbarten Plattenbereich eine Verengung bildet.

6. Knochenplatte nach einem der vorangehenden Ansprüche, **dadurch gekennzeichnet, dass** die Knochenschraube (6) und/oder die Platte (1) aus Titan besteht.

7. Knochenplatte nach einem der vorangehenden Ansprüche, **dadurch gekennzeichnet, dass** der Kopf (7) der Knochenschraube (6) ein rein konisches Außengewinde (9) aufweist, während das Innengewinde (5d) der Platte (1) rein zylindrisch ausgebildet ist.

8. Knochenplatte nach einem der vorangehenden Ansprüche, **dadurch gekennzeichnet, dass** der Gewindegrund des Knochengewindes der Knochenschraube (6) in der Nachbarschaft des Kopfes (7) einen sich, insbesondere konisch, erweiternden Bereich (12) aufweist und im Wesentlichen an den Gewindegrund des benachbarten Außengewindes (9) am Kopf (7) der Schraube (6) anschließt.

9. Knochenplatte nach einem der vorangehenden Ansprüche, **dadurch gekennzeichnet, dass** die Steigung des Feingewindes am Kopf (7) der Schraube kleiner ist als die Steigung des Knochengewindes (10) am Schaft der Schraube (6).

10. Knochenplatte nach einem der vorangehenden Ansprüche, dadurch gekennzeichnen, dass der Kopf (6) der Schraube (7) einen Innensechskant (11) aufweist.

## Claims

1. A bone plate with at least one screw for angularly stable fixation, in particular in the form of a hallux valgus plate, having at least one through-hole (2, 3, 4, 5) and at least one bone screw (6) comprising an external thread (9) in the head region, which thread tapers more severely in the screwing-in direction than a mating thread formed as an internal thread (5d) in the through-hole, such that, on screwing-in, the head (7) of the bone screw (6) becomes directionally stably wedged between the external thread (9) of the head (7) and the internal thread (5d) of the plate (1) to protect against rotation,
**characterised in that**
the bone plate (1) is configured resiliently in at least one region (2a; 2b; 3a; 3b; 4a; 4b) adjoining the through-hole (2, 3, 4, 5), such that the material of the bone plate (1) is not deformed into the plastic range by the expansion resulting from screwing in of the head (7) of the bone screw (6), such that, protection against rotation still being provided, over-expansion of the region surrounding the through-hole (2, 3, 4, 5) does not arise in any position of the bone screw (6), if it is screwed right through the plate (1), as a result of interaction of the external thread (9) of the head (7) of the bone screw (6) with the internal thread of the plate over the entire length of the thread (5d, 9).

2. A bone plate according to claim 1, **characterised in that** the region (2a, 2b, 3a, 3b, 4a, 4b) adjoining the through-hole (2, 3, 4, 5) is configured at least in part as a web or part of a ring.

3. A bone plate according to claim 2, **characterised in that** the region (2a, 2b, 3a, 3b, 4a, 4b) adjoining the through-hole (2, 3, 4, 5) comprises in plan view a substantially constant width or in section a substantially constant material cross-section, which is adjoined on both sides by a region of increasing web width, which however constitutes a constriction with respect to the plate in plan view.

4. A bone plate according to claim 3, **characterised in that** the region (2a, 2b, 3a, 3b, 4a, 4b) adjoining the through-hole (2, 3, 4, 5) consists of at least two regions of constant width, adjoined on both sides by a region of the plate with increased web width (2c, 2d, 3c, 3d, 4c, 4d, 5c).

5. A bone plate according to claim 2, **characterised in that** the region (2a, 2b, 3a, 3b, 4a, 4b) adjoining the through-hole (2, 3, 4, 5) is concentrated solely in the web-form or annular region which forms a constriction relative to the neighbouring plate region.

6. A bone plate according to any one of the preceding claims, **characterised in that** the bone screw (6) and/or the plate (1) consists of titanium.

7. A bone plate according to any one of the preceding claims, **characterised in that** the head (7) of the bone screw (6) comprises a purely conical external thread (9), while the internal thread (5d) of the plate (1) is purely cylindrical.

8. A bone plate according to any one of the preceding claims, **characterised in that** the thread root of the bone thread of the bone screw (6) comprises a region (12) which widens, in particular conically, in the vicinity of the head (7) and substantially adjoins the thread root of the neighbouring external thread (9) on the head (7) of the screw (6).

9. A bone plate according to any one of the preceding claims, **characterised in that** the pitch of the fine-pitch thread on the head (7) of the screw is smaller than the pitch of the bone thread (10) on the shank of the screw (6).

10. A bone plate according to any one of the preceding claims, **characterised in that** the head (7) of the screw (6) comprises a hexagon socket (11).

## Revendications

1. Lame osseuse présentant au moins une vis permettant une fixation à angle stable, en particulier en forme de lame Hallux-Valgus, avec au moins un trou traversant (2, 3, 4, 5) et au moins une vis d'os (6), qui présente dans la région de la tête un filet extérieur (9), qui se rétrécit plus fortement dans la direction de vissage qu'un filet opposé du trou traversant réalisé comme un filet intérieur (5d), de telle manière que la tête (7) de la vis d'os (6) se bloque en direction stable lors du vissage pour un blocage en rotation entre le filet extérieur (9) de la tête (7) et le filet intérieur (5d) de la lame (1), **caractérisée en ce que** la lame osseuse (1) est réalisée sous forme élastique, dans au moins une région (2a; 2b; 3a; 3b; 4a; 4b) adjacente au trou traversant (2, 3, 4, 5), de telle manière que la matière de la lame osseuse (1) ne se déforme pas dans le domaine plastique par la déformation résultant du vissage de la tête (7) de la vis d'os (6), de telle manière que, par l'interaction du filet extérieur (9) de la tête (7) de la vis d'os (6) avec le filet intérieur de la lame sur toute la longueur des filets (5d, 9) avec maintien du blocage en rotation, il ne se produise en aucune position de la vis d'os (6) une déformation excessive de la région entourant le trou traversant (2, 3, 4, 5), lorsque celle-ci est entièrement vissée à travers la lame (1).

2. Lame osseuse selon la revendication 1, **caractérisée en ce que** la région (2a, 2b, 3a, 3b, 4a, 4b) adjacente au trou traversant (2, 3, 4, 5) est configurée au moins en partie comme une nervure ou une partie d'une bague.

3. Lame osseuse selon la revendication 2, **caractérisée en ce que** la région (2a, 2b, 3a, 3b, 4a, 4b) adjacente au trou traversant (2, 3, 4, 5) présente dans une vue en plan une largeur essentiellement constante ou dans une vue en coupe une section transversale de matière essentiellement constante, à laquelle se raccorde de part et d'autre une région présentant une largeur de nervure croissante, qui représente cependant, dans la vue en plan, un étranglement par rapport à la lame.

4. Lame osseuse selon la revendication 3, **caractérisée en ce que** la région (2a, 2b, 3a, 3b, 4a, 4b) adjacente au trou traversant (2, 3, 4, 5) se compose au moins de deux régions de largeur constante, auxquelles se raccorde de part et d'autre une région de la lame présentant une largeur de nervure accrue (2c, 2d, 3c, 3d, 4c, 4d, 5c).

5. Lame osseuse selon la revendication 2, **caractérisée en ce que** la région (2a, 2b, 3a, 3b, 4a, 4b) adjacente au trou traversant (2, 3, 4, 5) se concentre exclusivement sur la région en forme de nervure ou de bague, qui forme un rétrécissement par rapport à la région de lame voisine.

6. Lame osseuse selon l'une quelconque des revendications précédentes, **caractérisée en ce que** la vis d'os (6) et/ou la lame (1) est constituée de titane.

7. Lame osseuse selon l'une quelconque des revendications précédentes, **caractérisée en ce que** la tête (7) de la vis d'os (6) présente un filet extérieur (9) purement conique, tandis que le filet intérieur (5d) de la lame (1) est de forme purement cylindrique.

8. Lame osseuse selon l'une quelconque des revendications précédentes, **caractérisée en ce que** le fond de filet du filet d'os de la vis d'os (6) présente, à proximité de la tête (7), une région (12) s'évasant, de préférence en cône, et se raccorde essentiellement au fond de filet du filet extérieur voisin (9) sur la tête (7) de la vis (6).

9. Lame osseuse selon l'une quelconque des revendications précédentes, **caractérisée en ce que** le pas du filet fin sur la tête (7) de la vis est plus petit que le pas du filet d'os (10) sur la tige de la vis (6).

10. Lame osseuse selon l'une quelconque des revendications précédentes, **caractérisée en ce que** la tête (7) de la vis (6) présente un hexagone à six pans intérieurs (11).
